# EUROPEAN PATENT APPLICATION

(11) **EP 0 549 078 A1**
(43) Date of publication of application: **30.06.1993**
(21) Application number: 92204073.8
(22) Date of filing: 23.12.1992
(51) Int. Cl.: A61F 2/30

(54) **Prosthesis for insertion in a long bone**

(30) Priority: 23.12.1991 NL 9102171
(71) Applicant: Sauter, Adolf Johan Marie, NL-2597 JT Den Haag (NL)
(72) Inventor: Sauter, Adolf Johan Marie, NL-2597 JT Den Haag (NL)
(74) Representative: Smulders, Theodorus A.H.J., Ir.

(57) **Abstract**

Joint prosthesis which can be fitted in a long bone (1), comprising a metal pin (10) ending at its upper end in a cone (13) for fixing thereto a spherical member which can be fitted in a joint socket of a patient. The prosthesis further comprises a supporting sleeve (15) of relatively short length in relation to the pin (10), which can be fixed in the proximal part of the long bone (1) and is provided with a hole (16), slot-shaped in section, which surrounds the metal pin (10) with space, whilst the pin (10) is equipped with carrying means (14) which can be supported by the upper edge (17) of the supporting sleeve (15).

## Description

This invention relates to a prosthesis comprising a metal pin which can be fitted in a long bone and ends at its upper end in a cone for fixing thereto a spherical member which can be fitted in a joint socket of a patient.

Such a prosthesis is known from practice and frequently used. Either the pin is fixed along its entire length in a long bone of a patient by means of a filler, such as acrylic cement, or it is made of such shape that it can be fixedly wedged in a long bone, without using a filler. A drawback of this known prosthesis is that the pin takes over the greater part of the bending forces of the long bone, so that the distribution of stress in the bone is seriously disturbed. This may lead to local bone resorption and loosening of the prosthesis. This drawback could be obviated by a prosthesis in which the pin to be fixed has the same elasticity modulus as the bone of an individual patient. It has been attempted to find a solution in the selection of the material of the pin. This presents a problem in that the choice of material can never be the appropriate choice for every individual patient.

The object of the invention is to provide a prosthesis that is capable of transmitting the bending forces to the long bone and in which the distribution of stress is therefore less adversely affected than in conventional prostheses. This is important for hip prostheses in particular, but it also holds for other prostheses that can be fixed in a long bone.

To that end, the prosthesis according to the invention is characterized in that it further comprises a supporting sleeve of short length in relation to the pin, which sleeve can be fixed in the proximal part of the long bone and is provided with a hole, slot-shaped in section, which surrounds the pin with space, whilst the pin is equipped with carrying means which can be supported by the upper edge of the supporting sleeve.

In this manner, the axial load applied to the prosthesis is taken up by the relatively short supporting sleeve, which transmits this load to the proximal part of the long bone that surrounds the supporting sleeve. The distal end of the pin is secured in the long bone so as to be movable in axial direction but fixed in radial direction. Because the supporting sleeve allows the pin to tilt in one direction and the distal end of the pin is retained in radial direction, the bending forces are transmitted direct onto the long bone surrounding the prosthesis. In this manner, under load, the long bone is subject to a physiological interplay of forces whereby bone resorption and loosening of the prosthesis are prevented.

For the retention of the carrying means in lateral direction, the upper edge of the supporting sleeve is provided with at least one bearing point for those carrying means. In order to obtain anatomically optimal positioning of the prosthesis, and hence a normal load pattern of the long bone, it is desired that the prosthesis can be fitted in several positions during surgery, both in lateral and in axial direction.

For fitting the prosthesis in different lateral positions (the varus and valgus positions), the upper edge of the supporting sleeve is preferably provided with three bearing points, located side by side, for the carrying means.

According to an alternative embodiment, the supporting sleeve, at the proximal end thereof, comprises a slide which can be shifted in transverse direction and fixed, and is provided with one bearing point for the carrying means.

For adjustment in axial direction, the carrying means can be fixed in the pin in different axial positions.

One embodiment of a prosthesis designed as a hip prosthesis in accordance with the invention will now be further explained, by way of example only, with reference to the accompanying drawings, in which:
Fig. 1 is a pin-shaped hip prosthesis according to the prior art, mounted in the long bone of the upper leg;
Fig. 2 is a front view of the hip prosthesis according to the invention, with the supporting sleeve shown partly in section;
Fig. 3 is a top plan view of the supporting sleeve, with the pin shown in section; and
Fig. 4 is the hip prosthesis according to the invention in mounted condition.

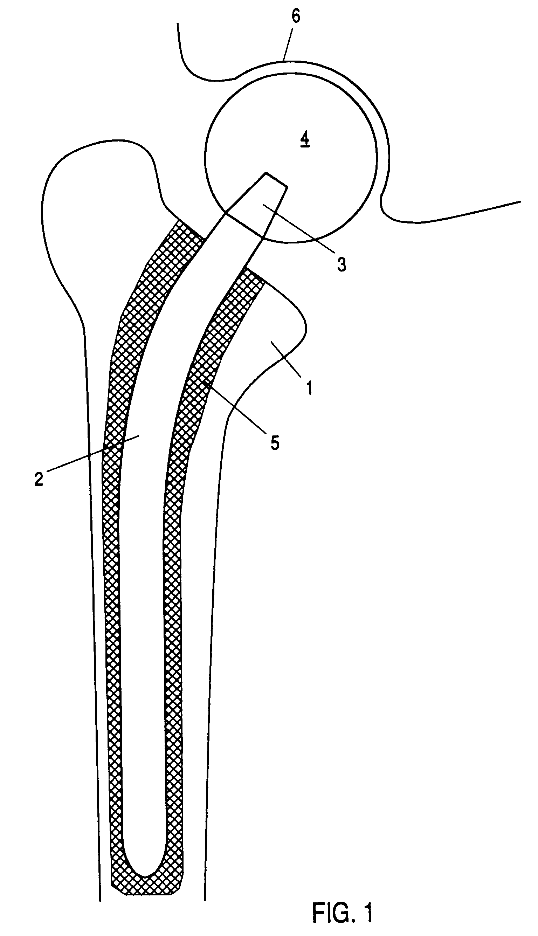

Fig. 1 shows a known, pin-shaped hip prosthesis, mounted in the proximal part of the long bone 1 of the upper leg. The hip prosthesis consists of a bent metal pin 2, provided at the upper end thereof with a cone 3 for fixing a ball 4 thereto. The ball 4 is located in the hip socket 6. The metal pin 2 is fixed along its entire length in the marrow cavity of the long bone 1 by means of acrylic cement 5. The metal pin 2 is therefore integral with the long bone 1 and transmits all the forces applied from the hip socket 6, both the vertical loads and the tilting moments, to the long bone 1.

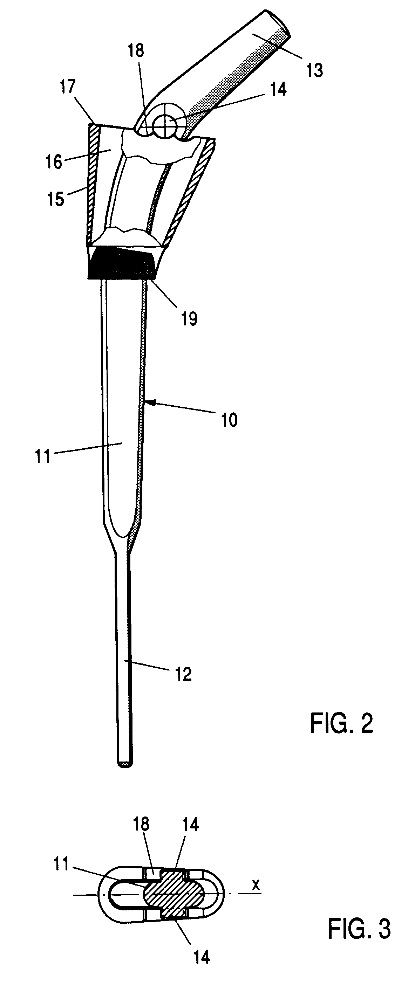

The hip prosthesis according to the invention is shown in Figs 2 and 3. The hip prosthesis consists of a bent, metal pin having a flat central portion 11 and an end 12, circular in section, whilst at the top of the pin 10 a cone 13 is present for mounting a ball thereon. Directly below the cone 13, the pin is provided with two carrying members designed as carrying journals 14 projecting laterally from the pin 10, on opposite sides thereof. These carrying journals 14 rest on a supporting sleeve 15, which can be fixed in the proximal part of the long bone and in which is provided a slotted hole 16, surrounding the flat central portion 11 of the pin 10 with ample clearance, in any case in lateral direction. The supporting sleeve 15 has a downwardly tapering shape, optionally with a bevelled lower end 19. The upper edge 17 of the supporting sleeve 15 is provided with bearing points 18, located side by side, for receiving therein the carrying journals 14 of the pin 10. Which of the three bearing points 18 is chosen by the orthopedic surgeon to mount the carrying journals 14 in depends on the patient's build and the position in which the supporting sleeve 15 is fixed in the proximal part of the long bone. The left-hand bearing point 18 is intended for mounting the hip prosthesis in the valgus position, the right-hand bearing point 18 for mounting it in the varus position.

One bearing point 18 for the carrying journals 14 will be sufficient if a slide (not shown) capable of sliding in the plane of the paper is arranged in the upper part of the supporting sleeve 15, which slide can be retained in any desired position. The displacement mechanism for this slide can consist of a screw spindle, bearing-mounted in the narrow sidewalls of the supporting sleeve 15. This variant is suitable in particular when carrying journals of relatively large diameter are used.

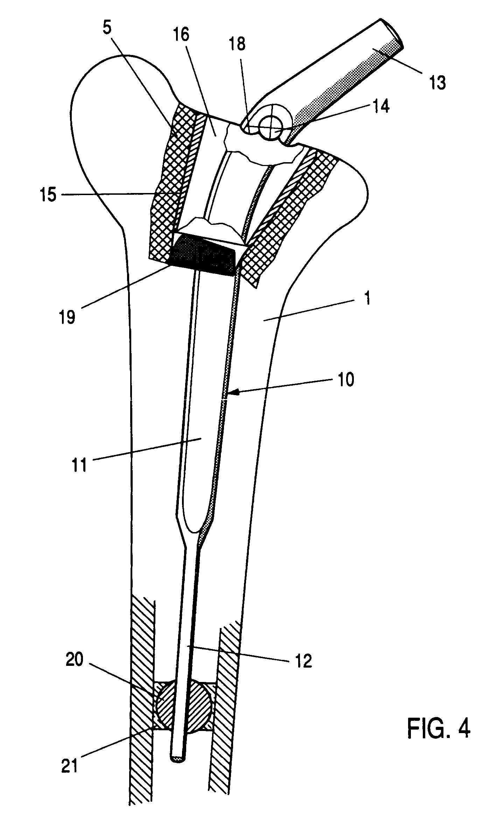

In the mounted condition of the hip prosthesis as shown in Fig. 4, the supporting sleeve 15 is fixed within the long bone 1 of the upper leg with acrylic cement 5. It is also possible, however, to give the supporting sleeve 15 an external shape such that it can be fixedly wedged in the long bone, without using acrylic cement 5. In that case, of course, one should dispose of a series of supporting sleeves 15 of different dimensions, from which the most suitable form for an individual patient can be selected.

The cylindrical lower end 12 of the pin 10 is mounted for axial movement in a plastic ball 20, which is rotatably retained in a case 21, fixedly mounted in the long bone 1.

The vertical load transmitted from the hip socket 6 (Fig. 1) to the prosthesis is taken up by the laterally confined carrying journals 14 resting on the upper edge 17 of the supporting sleeve 15. The joint consisting of supporting sleeve 15 and carrying journals 14 allows tilting motion of the pin 10 and therefore cannot take up any bending moments.

The bending moments transferred from the hip socket 6 to the prosthesis are transmitted to and taken up by a case 21 fixedly mounted in the long bone 1. The case 21 transmits the bending moments to the long bone 1 at that point, thereby providing for a normal load pattern of the long bone. When the supporting sleeve 15 has sunk into the long bone 1 after some time, which results in a reduction of the distance between the upper edge 17 of the sleeve 15 and the fixed case 21, the end 12 of the pin 10 can shift in axial direction relative to the ball 20, which is at the same time rotatable relative to the case 21. Accordingly, the supporting sleeve's sinking into the long bone 1 does not lead to loosening of the prosthesis, as with the hip prosthesis according to Fig. 1. Rotation of the ball 20 within the case 21 is further necessary for fitting the carrying journal 14 in different, lateral positions in one of the bearing points 18 or upon lateral displacement of the single bearing point 18 provided in a slide.

When the prosthesis according to the invention is used for other joints than the hip joint, such as the shoulder joint, the ankle joint, the wrist joint and the elbow joint, small adjustments are necessary within the framework of the basic principle of the invention.

For instance, in a prosthesis for one of these other joints, a substantially straight pin will be used, at any rate a pin that is bent to a lesser extent than the pin shown in Figs 2 and 4 for a hip joint prosthesis. Instead of being designed as a ball, the spherical element can be designed as a spherical dish, which can optionally serve as a carrying means that is matched directly with the supporting sleeve.

In that case, the bearing point in the carrying means has a shape adapted to the spherical dish.

## Claims

1. A prosthesis comprising a metal pin which can be fitted in a long bone and ends at its upper end in a cone for fixing thereto a spherical member which can be fitted in a joint socket of a patient, characterized in that the prosthesis further comprises a supporting sleeve (15) of short length in relation to the pin (10), which sleeve (15) can be fixed in the proximal part of the long bone (1) and is provided with a hole (16), slot-shaped in section, which surrounds the metal pin (10) with space, whilst the pin (10) is equipped with carrying means (14) which can be supported by the upper edge (17) of the supporting sleeve (15).

2. A prosthesis according to claim 1, characterized in that the carrying means (14) are provided adjacent the lower end of the cone (13) and project outside the pin (10) on opposite sides thereof, whilst the upper edge (17) of the supporting sleeve (15) comprises at least one bearing point (18) for the two carrying means (14).

3. A prosthesis according to claims 1-2, characterized in that the upper edge (17) of the supporting sleeve (15) comprises three bearing points (18) for the carrying means (14).

4. A prosthesis according to claims 1-2, characterized in that the supporting sleeve (15), at the proximal end thereof, is provided with a slide which can be moved in transverse direction and fixed in a desired position, the upper edge (17) of said slide comprising one bearing point (18).

5. A prosthesis according to any one of claims 1-4, characterized in that the carrying means (14) can be fixed in different axial positions in the pin (10).

6. A prosthesis according to any one of claims 1-5, characterized in that the hole (16), slot-shaped in section, provided in the supporting sleeve (15) extends transversely to the centreline of the carrying means (14).

7. A prosthesis according to any one of claims 1-6, characterized in that the supporting sleeve (15) has a tapering configuration in distal direction.

8. A prosthesis according to any one of claims 1-7, characterized in that it comprises a case (21) which can be fixed in the distal part of the long bone (1), said case (21) accommodating a ball (20) which is rotatable relative to the case (21), whilst said ball (20) is provided with a bore for receiving the end (12) of the pin (10) for axial movement in said bore.
